# EUROPEAN PATENT SPECIFICATION

(11) **EP 3 893 775 B1**
(45) Date of publication and mention of the grant of the patent: **17.07.2024**
(21) Application number: 20821361.1
(22) Date of filing: 23.11.2020
(51) Int. Cl.: A61B 17/435

(54) **CATHETER FOR THE TRANSFER OF HUMAN EMBRYOS WITH SECTION OF TRANSVERSE FOLDS AND ERGONOMIC HANDLE**
KATHETER FÜR DIE ÜBERTRAGUNG VON MENSCHLICHEN EMBRYONEN MIT EINEM ABSCHNITT VON QUERFALTEN UND EINEM ERGONOMISCHEN GRIFF
CATHÉTER POUR LE TRANSFERT D'EMBRYONS HUMAINS AVEC SECTION DE PLIS TRANSVERSAUX ET POIGNÉE ERGONOMIQUE

(30) Priority: 03.03.2020 GR 20200100117
(43) Date of publication of application: 20.10.2021
(73) Proprietor: Meridis, Eleftherios, 10673 Athens (GR)
(72) Inventor: Meridis, Eleftherios, 10673 Athens (GR)
(74) Representative: Modiano, Gabriella Diana
(86) International application number: PCT/GR2020/000061
(87) International publication number: WO 2021/176239

(56) References cited:
- WO-A2-2004/035110
- US-A- 5 195 979
- US-B1- 6 440 120

## Description

The present invention refers to a catheter for the transfer of embryos in the intrauterine cavity of a woman, an act that completes every in-vitro fertilization (IVF) treatment cycle for subfertile couples.

These catheters are familiar. The known catheters are consisted of two parts: an outer catheter (α)/ (guide) made of semi-rigid synthetic material, having a diameter of 5.5-6.6 fr and length of 16.7-17.3 cm and a lumen through which the inner catheter (β)/(soft) is introduced. The inner catheter is made of soft synthetic material, with a diameter of 2.8 fr and length of 23-24cm and has also a lumen.

The catheter (β) is the one which is used for "loading" of the embryos that are about to be transferred in the intrauterine cavity and is inserted inside catheter (α).

The catheter (α) is supporting catheter (β) due to being more rigid and assists it firstly in entering the cervical channel through the outer cervical os and secondly in performing the necessary maneuvers so that catheter (β) passes through the entire cervical channel and reaches the desired point inside the intrauterine cavity.

It becomes evident that an effective design of catheter (α) needs to serve two functions: firstly support of the softer catheter (β) since it will bend on its own when hold in hand, and secondly assistance of catheter (β) to approach and enter the outer cervical os and cervical channel , and to perform the necessary maneuvers by the doctor/clinician so that the catheter (β) is inserted deeper inside the cervical channel and the intrauterine cavity until the point where the embryo transfer will take place.

At this point is worth emphasizing on that the faster, smoother and as atraumatic as possible approach of the point inside the intrauterine cavity that the embryo transfer will take place, is decisive for the success of the whole IVF treatment cycle and achievement of embryo implantation and pregnancy. Any difficulty, delay, injury or micro-bleeding during the passage of the catheter (β) through the cervical channel can be catastrophic to the implantation process of the embryos.

However the existing catheters in the market have a disadvantage in achieving the above goal due to them being semi-rigid and straight in shape, the result being that on many occasions they approach the outer cervical os at an angle (especially in cases where the cervix protrudes obliquely in the vagina).Furthermore, the cervical channel is not shaped as a straight line but as a curve or sometimes even as the letter "S". This results to the fact that a standard catheter (α) being straight is unable to follow the shape and maneuver appropriately according to the shape of the cervical channel.

Many clinicians, in their attempt to navigate through the "S" shaped cervical channel with the existing catheters, bend with their fingers the end part of the outer catheter (α), aiming to give it a more anatomical design, that can deal with the curves of the cervical channel. Even so though, the catheter remains stable at the angle given to it by the doctor, resulting in it being unable to manage effectively the angles and curves of the cervical channel. In addition to that, the whole process becomes more difficult due to the fact that the catheter's handle is not ergonomically designed and doesn't have any reference points. A known catheter system for embryo transplantation is disclosed in US 5195979 A.

The invention is defined in appended claim 1.

The proposed catheter for the transfer of embryos has the following advantages
1. It can be autonomous flexible at a certain part of its length so that it can, and on its own navigate the shape of the cervical channel while passing through it, without delays or micro-injuries.
2. It can be bent, expanded, compressed and generally configured in the desired shape and angle by the operating clinician, in order to achieve easier, faster and without injuries passage through the cervical channel.
3. Makes possible a further adjustment of its angle and shape in case this is deemed necessary by the operating clinician.
4. The configuration of the catheter at the section of the transverse folds is achieved without narrowing the lumen of catheter (α) as to affect the functionality of catheter (β).

The proposed catheter consists of an outer, harder catheter 18.5 cm long (α) and an inner soft catheter 24.5 cm long (β).

At a distance between 5 and 6 cm from its tip, catheter (α) has an element of transverse folds (1) in the shape of an "accordion". The distance of the center of the folds is proposed at 5.5cm and it can vary +/- 1 cm in variations of the same proposal. These folds can expand, or compress, bend and finally be configured in the desired angle without narrowing the lumen of catheter (α) and affecting the functionality of catheter (β).

At a distance of 1,2,3,4 and 5cm from the tip of catheter (α) there are indicative dots (2) that can assist the clinician know, how far has the catheter been inserted inside the cervical channel.

The catheter (β) is consistent in configuration, soft, flexible and with a diameter that allows it to be inserted in the lumen of catheter (α). It too has a lumen that is used for the loading, carriage and transfer of the embryos.

The base/handle of catheter (α) internally is shaped as a crucible (3), so that it facilitates the entry of catheter (β) and subsequent assembly of the bases/handles of the two catheters. The base/handle of catheter (β) is too shaped as a crucible (4) so to connect firmly with the base /handle of catheter (α).

When catheters (α) and (β) are assembled, their bases externally form a handle of elliptical shape, 1.3 cm wide, 2cm high and 1.1 cm thick. The handle presents with increased thickness perimetrically and reduced centrally thus achieving a larger and more comfortable surface for contact with the operator's hand. This characteristic permits a more accurate and steadier assembly and separation of catheters (α) and (β) by rotation of the two bases (3,4) in opposite directions.

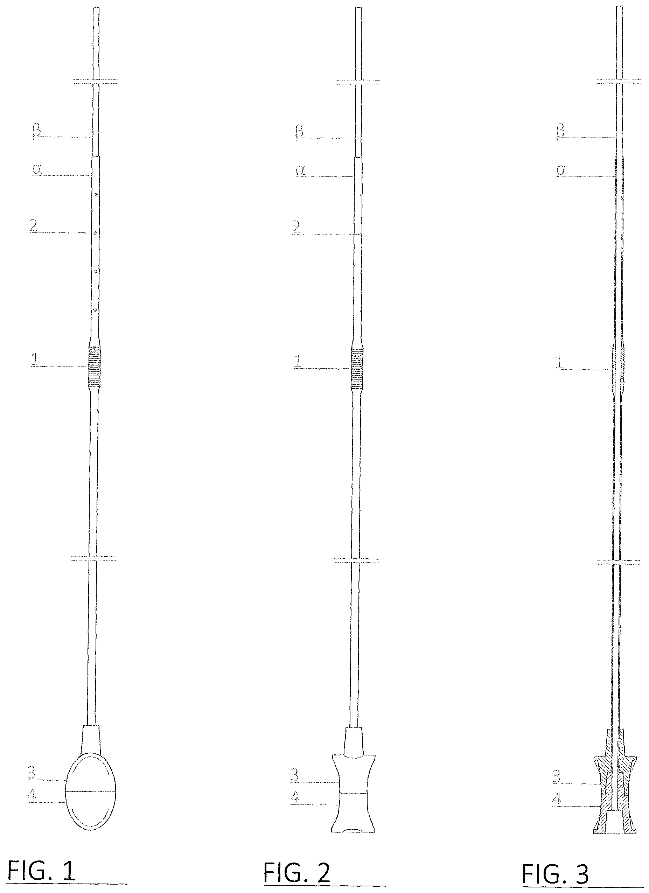

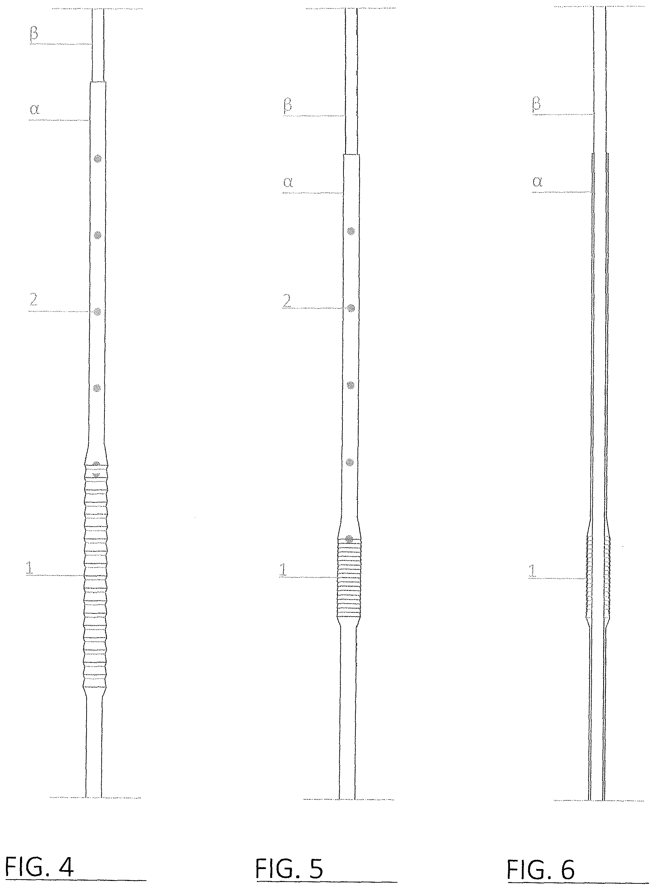

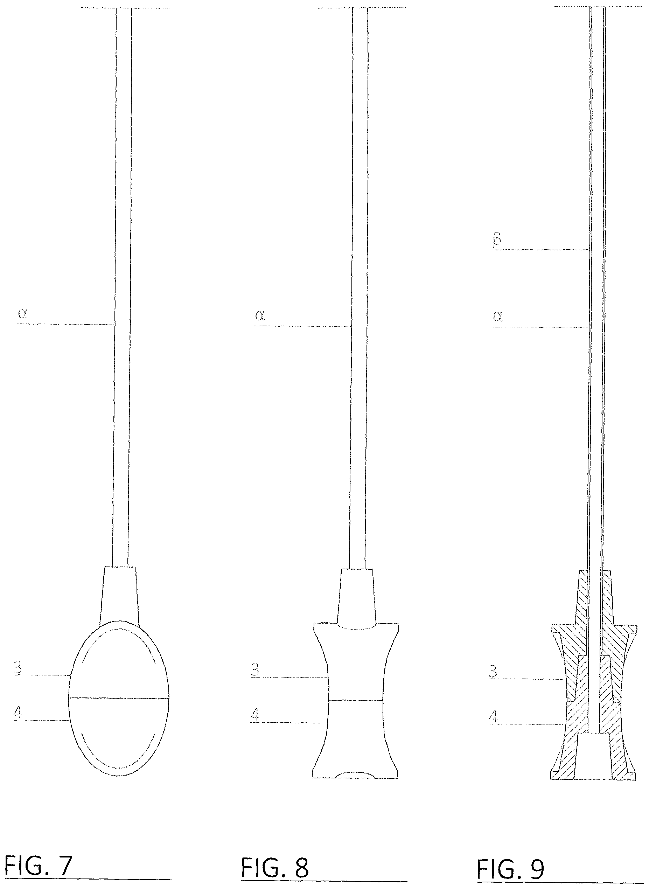

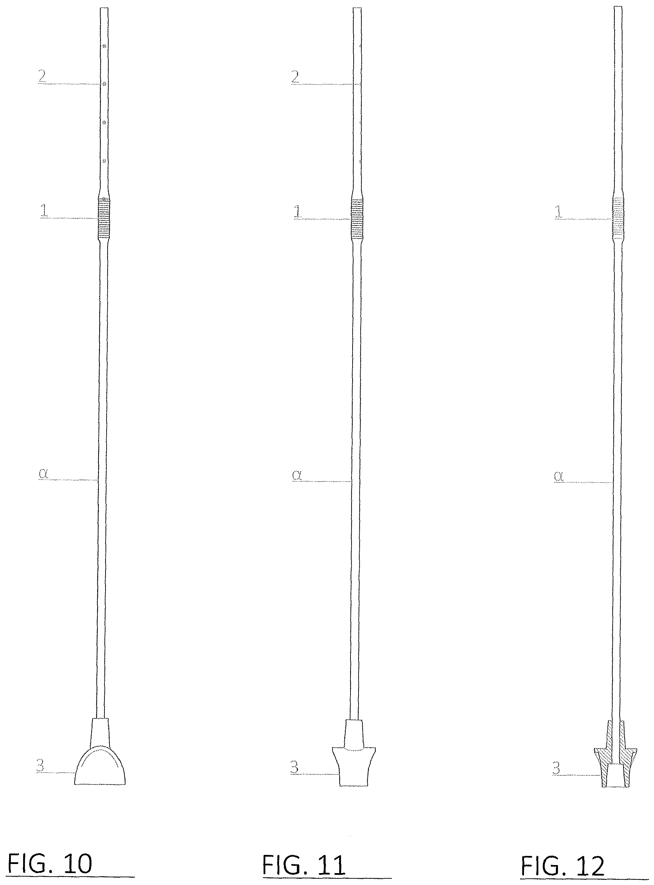

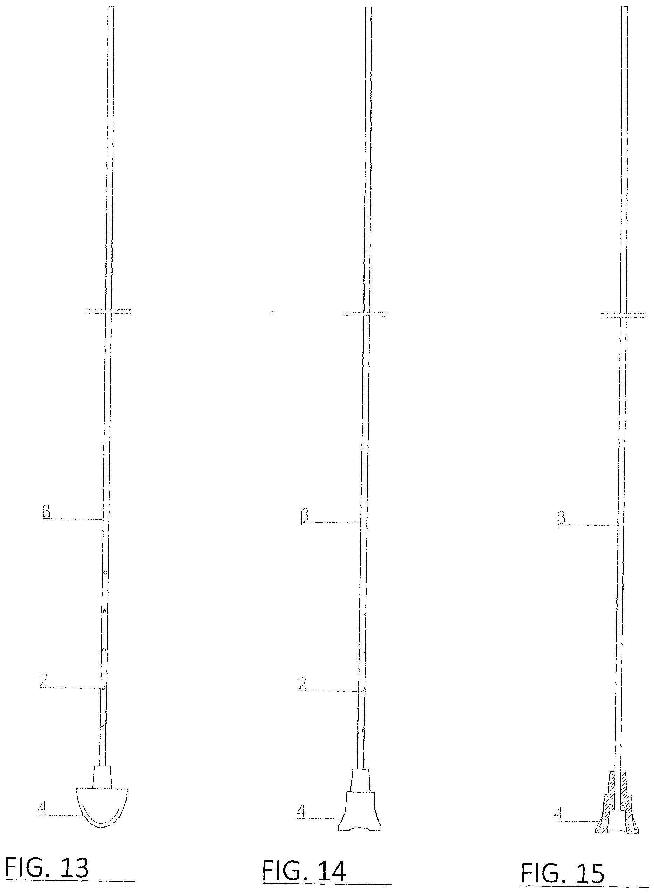

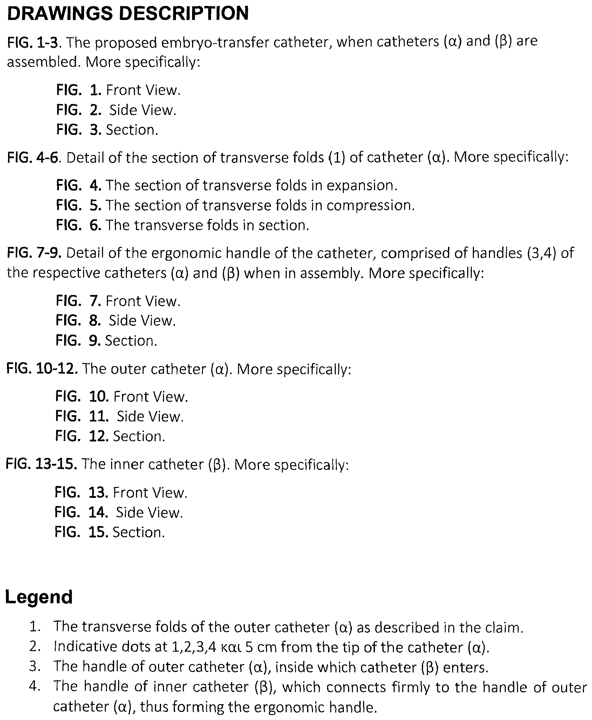

## Claims

1. Catheter for the transfer of human embryos, wherein the catheter comprises an outer, harder catheter (α) and an inner, soft catheter (β), **characterized in that** outer catheter (α) has element of transverse folds (1) in the shape of an "accordion". such as when being compact, the element of transverse folds is 1 cm long while the distance of its center from the tip of the catheter is 5.5 cm but can vary +/- 1 cm in variations of the same design.

2. Catheter according to claim 1, wherein the element of transverse folds (1) has the ability to bend, expand, compress and generally be configured in the desired shape and angle by the operating clinician in order to facilitate its passage through the cervical channel.

3. Catheter according to claim 2, wherein said shape and angle are capable of being reconfigured by the operating clinician if deemed necessary.

4. Catheter according to any one of claims 1 to 3, wherein the element of transverse folds (1) is also capable of automatically adjusting in shape and angle during its passage through the cervix, following the anatomy of the cervical channel.

5. Catheter according to any one of claims 1 to 4, **characterized in that**
a) it is flexible at a specific part, so that it can automatically and on its own successfully adjust in shape and angle according to the shape of the cervical channel and intrauterine cavity without causing delay or micro-injuries;
b) it can be configured to and maintain a shape and angle given to it by the operating clinician in order to achieve an easier, quicker and more atraumatic entry and passage through the cervical channel;
c) it makes feasible a further re-configuration of its shape and angle if deemed necessarys;
d) a configuration of its shape and angle is achieved without narrowing the lumen of catheter (α) as to affect the functionality of catheter (β).

## Patentansprüche

1. Katheter für die Übertragung menschlicher Embryonen, wobei der Katheter einen äußeren, härteren Katheter (α) und einen inneren, weichen Katheter (β) umfasst, **dadurch gekennzeichnet, dass** der äußere Katheter (α) ein Element mit Querfalten (1) in Form eines "Akkordeons" hat, derart, dass im kompakten Zustand das Element mit Querfalten 1 cm lang ist, während der Abstand zwischen seiner Mitte und der Spitze des Katheters 5,5 cm beträgt, aber in Variationen desselben Designs um +/- 1 cm schwanken kann.

2. Katheter gemäß Anspruch 1, worin das Element mit Querfalten (1) die Fähigkeit hat, sich zu biegen, zu expandieren, zu komprimieren und allgemein vom operierenden Kliniker in der gewünschten Form und dem gewünschten Winkel konfiguriert zu werden, um seine Passage durch den Zervixkanal zu erleichtern.

3. Katheter gemäß Anspruch 2, wobei die Form und der Winkel vom operierenden Kliniker bei Bedarf neu konfiguriert werden können.

4. Katheter gemäß einem beliebigen der Ansprüche 1 bis 3, wobei das Element mit Querfalten (1) auch in der Lage ist, sich während seiner Passage durch die Zervix, der Anatomie des Zervixkanals folgend, automatisch in Form und Winkel anzupassen.

5. Katheter gemäß einem beliebigen der Ansprüche 1 bis 4, **dadurch gekennzeichnet, dass**
a) er an einem bestimmten Teil biegsam ist, so dass er sich automatisch und eigenständig erfolgreich in Form und Winkel entsprechend der Form des Zervixkanals und der intrauterinen Höhle anpassen kann, ohne Verzögerung oder Mikroverletzungen auszulösen;
b) er konfiguriert werden kann, um eine Form und einen Winkel beizubehalten, die ihm vom operierenden Kliniker verliehen wurden, um einen leichteren, schnelleren und atraumatischeren Eintritt und eine ebensolche Passage durch den Zervixkanal zu erreichen;
c) er eine weitere Neukonfiguration seiner Form und seines Winkels ermöglicht, wenn dies als notwendig erachtet wird;
d) eine Konfiguration seiner Form und seines Winkels erzielt wird ohne Verengung des Lumens des Katheters (α), so dass die Funktionalität des Katheters (β) beeinträchtigt würde.

## Revendications

1. Cathéter pour le transfert d'embryons humains, dans lequel le cathéter comprend un cathéter extérieur, plus dur (α) et un cathéter intérieur, plus souple (β), **caractérisé en ce que** le cathéter extérieur (α) a un élément de plis transversaux (1) sous la forme d'un « accordéon » de sorte que, lorsqu'il est compact, l'élément de plis transversaux mesure 1 cm de long tandis que la distance de son centre à l'extrémité du cathéter est de 5,5 cm mais peut varier de +/- 1 cm dans des variantes du même modèle.

2. Cathéter selon la revendication 1, dans lequel l'élément de plis transversaux (1) a la capacité de fléchir, s'élargir, se comprimer et généralement d'être configuré selon la forme et l'angle voulus par le clinicien opérateur afin de faciliter son passage à travers le canal cervical.

3. Cathéter selon la revendication 1, dans lequel lesdits forme et angle peuvent être reconfigurés par le clinicien opérateur si nécessaire.

4. Cathéter selon l'une quelconque des revendications 1 à 3, dans lequel l'élément de plis transversaux (1) est également capable d'ajuster automatiquement sa forme et son angle durant son passage à travers le col utérin, en suivant l'anatomie du canal cervical.

5. Cathéter selon l'une quelconque des revendications 1 à 4, **caractérisé en ce que**
a) il est flexible au niveau d'une partie spécifique, de sorte qu'il peut automatiquement ajuster sa forme et son angle en fonction de la forme du canal cervical et de la cavité intra-utérine sans provoquer de délai ou de microlésions ;
b) il peut être configuré et conserver la forme et l'angle qui lui sont donnés par le clinicien opérateur afin de permettre une entrée et un passage plus faciles, plus rapides et plus atraumatiques à travers le canal cervical ;
c) il permet une reconfiguration de sa forme et de son angle si nécessaire ;
d) une configuration de sa forme et de son angle est obtenue sans rétrécir la lumière du cathéter (α) au point d'affecter la fonctionnalité du cathéter (β).
